# EUROPEAN PATENT SPECIFICATION

(11) **EP 3 634 491 B1**
(45) Date of publication and mention of the grant of the patent: **05.04.2023**
(21) Application number: 18711330.3
(22) Date of filing: 14.03.2018
(51) Int. Cl.: A61K 47/36, A61K 47/46

(54) **NATURAL AND ORGANIC BINDER COMPOSITIONS FOR ORAL SOLID DOSAGE FORMS**
NATÜRLICHE UND ORGANISCHE BINDEMITTELZUSAMMENSETZUNGEN FÜR ORALE FESTE DOSIERUNGSFORMEN
COMPOSITIONS DE LIANT ORGANIQUES NATURELLES POUR DES FORMES PHARMACEUTIQUES SOLIDES ORALES

(30) Priority: 06.06.2017 EP 17174622
(43) Date of publication of application: 15.04.2020
(73) Proprietor: BioGrund GmbH, 65510 Hünstetten (DE)
(72) Inventor: SPECHT, Felix, Ashburn, Virginia 20147 (US); YUNIS, Mahmud, 65203 Wiesbaden (DE)
(74) Representative: Uexküll & Stolberg
(86) International application number: PCT/EP2018/056422
(87) International publication number: WO 2018/224191

(56) References cited:
- R. GOSWAMI ET AL: "Natural gums and its pharmaceutical application", EUROPEAN JOURNAL OF ENDOCRINOLOGY, vol. 3, no. 1, 23 February 2014 (2014-02-23), pages 112-121, XP055348405, GB ISSN: 0804-4643, DOI: 10.1530/acta.0.1260474
- Patel Shailendra ET AL: "Natural Binding Agents in Tablet Formulation", International Journal of Pharmaceutical & Biological Archives, 1 January 2012 (2012-01-01), pages 466-473, XP055403443, Retrieved from the Internet: URL:http://ijpba.info/ijpba/index.php/ijpb a/article/viewFile/653/438
- Jo Goossens ET AL: "59th Starch Convention 2008", Starch - Stärke, 1 February 2008 (2008-02-01), pages 59-60, XP55468693, DOI: 10.1002/star.200890011 Retrieved from the Internet: URL:onlinelibrary.wiley.com/doi/10.1002/st ar.200890053/pdf
- Anonymous: "Remington: The Science and Practice of Pharmacy", 2000, Williams&Wilkins, XP002776446, page 860

## Description

### FIELD OF THE INVENTION

The invention relates natural binding compositions for use in oral solid dosage forms and to a method for their production. The invention also relates to a solid oral dosage form comprising said natural binder composition. More particularly, the invention pertains to providing cohesiveness on the ingredients of solid dosage forms to form granules or tablets by natural binder compositions in the form of a pre-mix. This ensures the solid dosage form remains intact after processing and a clean label may be claimed for the finished products.

### DESCRIPTION OF RELATED ART

In past years, as consumer awareness in health and natural foods has increased, the utilization of natural additives instead of artificial additives in the food and pharma industry has progressively increased, and the variety of usage thereof also has continued to gain interest. In terms of consumer preference, as it relates to pharmaceutical dosage forms and health functional foods, wellbeing, health and ecologically friendly factors in manufacturing processes are reflected more and more important, and consequently many products comprised of natural components are being established. Consequently, the market share of these naturally constituted products is also growing. In recent years, there has been an incredible growth in natural product developments, which are essential to be used for a variety of purposes.

R. Goswami et al. "Natural gums and its pharmaceutical application", European Journal of Endocrinology, Vol. 3, No. 1, pages 112-121 the pharmaceutical application of certain natural gums as binders in tablet formulations.

Nevertheless, it is understood that efforts at formulating all-natural or substantially all-natural nutritional supplements in a solid form contained exclusively or almost exclusively certified organic/naturally ingredients have not been successful so far. In particular, these developments have not lead tablets ensuring reliable characteristics, like acceptable tablet hardness, good friability and/or acceptable dissolution profile of the active ingredient. To the contrary, the use of natural ingredients can result in adverse processing characteristics, such as reduced flowability, poor compressibility of the powder blend and batch to batch variation of finished product.

In the pharmaceutical and food industries, a number of excipients are used to manufacture solid dosage forms. These excipients are widely involved in the improvement of flowability, compression characteristics, binding and disintegration properties for enhancing the hardness of tablets or enhancing the process capability of solid dosage forms. The usage of excipients in solid dosage formulations was to perform as inert substance to deliver the necessary dosage, uniformity and volume for the precise administration of the active ingredient, but in current solid dosage forms they often provide multi-functional roles such as enhancing the bioavailability/solubility of the active ingredient, improving of patient acceptability and ensuring effectiveness of manufacture.

Tablets are the most accepted dosage form among pharmaceutical/nutritional product. Tablets are manufactured by compressing a suitable powder mixture in a die at high compression force. The powder mixture contains besides the active usually also fillers, disintegrants, lubricants, glidants and binders. The industrial production of high quality tablets involves a tablet mixture with good properties regarding homogeneity, flowability and compressibility. If the powder mixture does not have these properties it has to be pre-processed i.e. by granulation, otherwise direct compression can be utilized.

Two main methods of manufacturing tablets are known:
**Direct compression:** the powder mixture is mixed during a period of time and can directly be compressed into tablets. This method is seen as the most efficient and desirable method. The choice of binders is extremely critical for direct compression tablets.

**Granulation techniques:** consist of wet granulation and dry granulation/slugging methods where binding agents are added in liquid form and in dry form respectively. Some actives exhibit poor fluidity and compressibility and in those case granules with acceptable flow and compression properties have to be formed.

Binders are added to the tablet formulation to provide plasticity as well as increased inter-particle bonding strength and hardness in the tablet. Binders are substances that promote cohesiveness between the ingredients and keep the ingredients together to form the final tablet. The systematic scanning of new natural materials for potential use as binding agent in solid dosage formulations continues to be of interest for the industry. This is for the reason that different binding agents or mixtures of binding agents can be beneficial in succeeding in various mechanical strength and active release properties for different applications. The right choice of the binder or of the binder mixture for the development of solid dosage forms requires broad knowledge of binder properties for enhancing the robustness of the formulation and also of the inter-particle forces between the various materials constituting the solid dosage form, particularly tablets.

To the knowledge of the inventor, there have not been any described or commercially existing natural binder systems in powder forms available that are comprised entirely or almost entirely of natural/certified organic components prior to this invention. And therefore, there is a requirement for improved binding agents comprised entirely or almost entirely of natural/ certified organic ingredients. Existing artificial binders are: Polyvinyl Pyrrolidone (PVP), Polyethylene Glycols (PEG), modified Cellulose, Pregelatinized Starch etc. Recent trend towards the use of natural and non-toxic materials requires the substitution of the existing synthetic additives with natural ones.

### SUMMARY OF THE INVENTION

The invention is set out in the appended claims.

In one embodiment of the present invention, a natural binder composition for use either in direct compression or granulation techniques is provided. Preferably, the binder is present in powder form. The natural binder composition according to one embodiment of the present invention comprises a blend of components a. to d. selected from a. 5% to 65% by weight of guar gum, xanthan gum, or a combination thereof; b. 5% to 65% by weight of gum arabic; c. 5% to 65% by weight pullulan, honey powder, agave powder, or isolated pea protein or any combination of the foregoing; and d. 1% to 15% by weight natural plant fibers, all weight percentages being based on the dry weight of the binder composition.

**Guar Gum:** also known as guaran, is a substance made from guar beans which has thickening and stabilizing properties useful in various industries, traditionally the food industry. The guar seeds are dehusked, milled and screened to obtain the guar gum. It is typically produced as a free-flowing, off-white powder. It is classed as a galactomannan. In Europe, guar gum has EU food additive code E412.

**Xanthan Gum:** also known as xanthan, is a polysaccharide produced from a range of simple sugars using a fermentation process. In Europe, guar gum has EU food additive code E415.

**Gum Arabic:** also known as acacia gum, is a natural gum consisting of the hardened sap of various species of the acacia tree. Gum Arabic is a complex mixture of glycoproteins and polysaccharides. It is the original source of the sugars arabinose and ribose, both of which were first discovered and isolated from it, and are named after it. It is edible and has E number E414.

**Pullulan:** is a polysaccharide edible, mostly tasteless polymer consisting of maltotriose units. As a food additive, it is known by the E number E1204.

**Honey powder:** As an alternative to liquid honey for certain food applications, honey powder, based on honey with a carrier (like maltodextrin, gluten-free, GMO-free IP-certified maltodextrin or further carrier) and produced without any further additive. The final form is a free-flowing powder with a fine to coarse crystalline structure and a light yellow to pale beige colour. While its odour is mild and practically neutral, its taste offers the typical honey sweetness with a light aromatic note to honey.

**Agave powder:** is agave syrup with a carrier (like maltodextrin or cellulosic carrier or further carrier).

**Isolated pea protein:** is a natural, vegetable raw material with high nutritional properties. It offers an extraordinary amino acids profile than whey protein. Therefore it is a wholesome protein that is particularly suitable for vegetarians and those with allergy against lactose and gluten. It is commercially available e.g. under the trademark name "ERBOTIN^{®}".

Preferred **natural fibers** are selected from: apple fiber, cellulose powder, dehydrated fruit powder, rice powder or rice extract and mixtures thereof. Preferred dehydrated fruit powders include banana powder, apple powder, pear powder, blueberry powder, cherry powder, cranberry powder, grape powder, peach powder, strawberry powder, raspberry powder and mixtures of the foregoing. Preferred powder sizes are 8 Mesh (Thru USS#8: 90% minimum; thru USS#30: 25% maximum) and 35 Mesh: Thru USS#35: 90% minimum. It is further preferred that untreated powders are used, although calcium stearate may be added at 0.5% maximum to prevent caking. In certain cases the natural fibers may contain sodium sulfite up 600 ppm or SO₂ to inhibit enzymatic and non-enzymatic browning. Rice extract is also known as red yeast rice extract and is a substance that has been extracted from rice that has been fermented by yeast.

In another embodiment the present invention provides a method for producing a natural binder composition wherein in a blend of components a. to d. selected from: a. 5% to 65% by weight guar gum, xanthan gum or a combination thereof; b. 5% to 65% by weight gum arabic; c. c. 5% to 65% by weight pullulan, honey powder, agave powder, or isolated pea protein or any combination of the foregoing; and d. 1% to 15% by weight natural plant fibers, all weight percentages being based on the dry weight of the binder composition, is provided and physically blended until a homogeneous mixture is attained. The method is conducted without carrying out any chemical reaction process.

It has been found that the natural binder composition according to the present invention improves the flowability and compressibility of the whole tableting powder mixture and ensures efficient manufacturing of tablets of a consistent weight, uniform strength and batch to batch consistency. Preferably the natural binder composition according to the present invention is used in tablets in amounts ranging from 1 to 60 % w/w based on the total weight of the tablet in the tablet formulation (on a dry weight basis), more preferably ranging from 1 to 40 % w/w. In particular, the invention has a positive influence on tablet hardness: already 3% content of the inventive composition in the formulation results higher values than tablets with the same (or even higher) content of a dry binding agent like hydroxypropyl cellulose. Due to this fact the invention is an excellent natural/organic binder with good properties and also as potential substitute for conventional dry binders.

### DETAILED DESCRIPTION OF THE INVENTION

In accordance with an embodiment of the present invention, the natural binder composition comprises a blend of components selected from: a. of about 5% to about 65% by weight guar gum, xanthan gum or a combination thereof; b. of about 5% to about 65% by weight gum arabic; of about 5% to about 65% by weight pullulan, honey powder, agave powder, or isolated pea protein or any combination of the foregoing; and d. about 1% to about 15% by weight natural plant fibers, all weight percentages being based on the dry weight of the binder composition.

According to another embodiment of the present invention, the natural binder composition consists of a blend of components selected from: a. of about 5% to about 65% by weight guar gum, xanthan gum or a combination thereof; b. of about 5% to about 65% by weight gum arabic; of about 5% to about 65% by weight pullulan, honey powder, agave powder, or isolated pea protein or any combination of the foregoing; and d. about 1% to about 15% by weight natural plant fibers, all weight percentages being based on the dry weight of the binder composition.

The natural binder composition may be used in tablets in amounts prefereably ranging from about 1 to about 60 % w/w, more preferably from about 1 to about 40% w/w; most preferably from about 3 to about 30% w/w based on the total weight of the tablet (on a dry weight basis).

The amount of pullulan, honey powder, agave powder, or isolated pea protein or any combination of the foregoing, which serves as a primary natural binder in the binder composition according to the present invention, is within the range of about 5% to about 65% by weight of the binder composition (on a dry weight basis). The grade of the pullulan can be organic certified and/or according to USP/NF. An amount ranging from about 20% to about 40% by weight of the binder composition (on a dry weight basis) is preferred.

The amount of gum arabic which serves as a secondary natural binder in the binder composition according to the present invention, is within the range of about 5% to about 65% by weight of the binder composition (on a dry weight basis). The grade of the gum arabic can be organic certified and/or according to Food Chemical Codex (FCC). An amount ranging from about 20% to about 45% by weight of the binder composition (on a dry weight basis) is preferred.

The amount of guar gum, xanthan gum or combination thereof which serves as a third natural binder in the binder composition according to the present invention, is within the range of about 5% to about 65% by weight of the binder composition (on a dry weight basis). The grade of the guar gum or xanthan gum can be organic certified and/or according to Food Chemical Codex (FCC). An amount ranging from about 20% to about 40% by weight of the binder composition (on a dry weight basis) is preferred.

A plant fiber as defined above is added as a natural flow aid into the compound according to the present invention in an amount within a range of about 1% to about 15% by weight of the binder composition (on a dry weight basis). The grade of the fiber can be organic certified or conventional. An amount ranging from about 2% to about 8% by weight of the binder composition (on a dry weight basis) is preferred.

According to another embodiment of the present invention a method for producing a natural binder composition is provided where in a blend of components a. to d. selected from: a. 5% to 65% by weight guar gum, xanthan gum or a combination thereof; b. 5% to 65% by weight gum arabic; c. 5% to 65% by weight pullulan, honey powder, agave powder, or isolated pea protein or any combination of the foregoing; and d. 1% to 15% by weight natural plant fibers, all weight percentages being based on the dry weight of the binder composition, is provided and physically blended until a homogenous mixture is attained. The blender is preferably a V-type powder blender, but may also be any other blender suitable for blending dry powders.

Optionally the dry powder blend may further be granulated by slowly adding sufficient water until granules are formed, and then sieving and drying the so-obtained granules. Granulation is preferably carried out in a planetary mixer. The granulation is intended to enhance the flowability and compressibility of the dry powder blend, and is non-dusting. Other granulation methods can be applied instead, such as spray granulation or roller compaction.

According to a still further embodiment, the present invention provides a solid dosage form formulation containing the binder composition of claim 1 in an amount ranging from about 1% to about 60%, preferably about 1% to about 40%, more preferably in a range from about 2% to about 20%, such as about 3% to about 5% w/w based on the total weight of the tablet in the tablet formulation (on a dry weight basis).

Hereinafter, the invention will be described in detail with reference to the following examples.

### EXAMPLES

The following examples 1-15 illustrate the invention. All percentages are by weight of the composition.

### Example 1:

| **Excipient:** | **Grades:** | **% w/w** |
|---|---|---|
| Guar Gum | 21 CFR 184.1339 / organic | 30 |
| Gum Arabic | FCC / organic | 35 |
| Pullulan | USP / NF / organic | 30 |
| Apple Fiber | Organic | 5 |

A dry binder composition was produced by mixing of the ingredients in a V-type powder blender until a homogenous mixture was attained. The dry powder blend was also granulated in a planetary mixer by slowly adding sufficient water until granules were formed. The granules were then sieved through a 1-2 mm sieve and dried in an oven at 30°C until the moisture content of the granules was lower than 5%. The dried granules were sieved again trough the same sieve.

In the following examples the dry binder compositions were produced as in Example 1, however with different ingredients or different amounts per weight of the ingredients as in Example 1:

### Example 2:

| **Excipient:** | **Grades:** | **% w/w** |
|---|---|---|
| Guar Gum | 21 CFR 184.1339 / organic | 25 |
| Gum Arabic | FCC / organic | 35 |
| Pullulan | USP / NF / organic | 30 |
| Apple Fiber | organic | 10 |

### Example 3:

| **Excipient:** | **Grades:** | **% w/w** |
|---|---|---|
| Guar Gum | 21 CFR 184.1339 / organic | 30 |
| Gum Arabic | FCC / organic | 35 |
| Pullulan | USP / NF / organic | 30 |
| Cellulose Powder | FCC | 5 |

### Example 4:

| **Excipient:** | **Grades:** | **% w/w** |
|---|---|---|
| Guar Gum | 21 CFR 184.1339 / organic | 30 |
| Gum Arabic | FCC / organic | 35 |
| Pullulan | USP / NF / organic | 30 |
| Banana Powder | organic | 5 |

### Example 5:

| **Excipient:** | **Grades:** | **% w/w** |
|---|---|---|
| Guar Gum | 21 CFR 184.1339 / organic | 30 |
| Gum Arabic | FCC / organic | 35 |
| Pullulan | USP / NF / organic | 30 |
| Rice Extract | FCC / organic | 5 |

### Example 6:

| **Excipient:** | **Grades:** | **% w/w** |
|---|---|---|
| Guar Gum | 21 CFR 184.1339 / organic | 35 |
| Gum Arabic | FCC / organic | 35 |
| Pullulan | USP / NF / organic | 20 |
| Apple Fiber | organic | 10 |

### Example 7:

| **Excipient:** | **Grades:** | **% w/w** |
|---|---|---|
| Guar Gum | 21 CFR 184.1339 / organic | 40 |
| Gum Arabic | FCC / organic | 25 |
| Pullulan | USP / NF / organic | 30 |
| Rice Concentrate | FCC / organic | 5 |

### Example 8:

| **Excipient:** | **Grades:** | **% w/w** |
|---|---|---|
| Guar Gum | 21 CFR 184.1339 / organic | 30 |
| Gum Arabic | FCC / organic | 35 |
| Pullulan | USP / NF / organic | 30 |
| Rice Extract | FCC / organic | 5 |

### Example 9:

| **Excipient:** | **Grades:** | **% w/w** |
|---|---|---|
| Guar Gum | 21 CFR 184.1339 / organic | 30 |
| Gum Arabic | FCC / organic | 30 |
| Pullulan | USP / NF / organic | 30 |
| Rice Concentrate | FCC / organic | 10 |

### Example 10:

| **Excipient:** | **Grades:** | **% w/w** |
|---|---|---|
| Guar Gum | 21 CFR 184.1339 / organic | 25 |
| Gum Arabic | FCC / organic | 35 |
| Pullulan | USP / NF / organic | 30 |
| Banana Powder | FCC / organic | 10 |

### Example 11

A test series was defined to validate the influence of the invention on tablet hardness. Several formulations with different binder contents were investigated with respect to the tablet hardness they provide.

Tablets were compressed with a standard round tooling with 10 mm diameter, 5 mm thickness and engraving. For the characterization, the tablets were tested with respect to tablet hardness (according to Ph. Eur. 2.9.8).

The following tables 1-5 illustrate the formulations prepared. All percentages are by weight of the composition.

Binder Formulation 1 (Comparative): with 3% HPC

| **Ingredient:** | **% w/w** |
|---|---|
| Microcrystalline Cellulose | 66,9 |
| Dicalciumphosphate | 29,1 |
| Magnesium Stearate | 1 |
| HPC | 3 |

Binder Formulation 2 (Comparative): with 5% HPC

| **Ingredient:** | **% w/w** |
|---|---|
| Microcrystalline Cellulose | 65,5 |
| Dicalciumphosphate | 28,5 |
| Magnesium Stearate | 1 |
| HPC | 5 |

Binder Formulation 3 (Inventive): with 3% Natural Binder Pre-Mix

| **Ingredient:** | **% w/w** |
|---|---|
| Microcrystalline Cellulose | 66,9 |
| Dicalciumphosphate | 29,1 |
| Magnesium Stearate | 1 |
| Invented Binder Pre-Mix | 3 |

In the following examples the dry binder compositions were produced as in Example 1, however with different ingredients or different amounts per weight of the ingredients as in Example 1:

### Example 12

| **Excipient:** | **Grades:** | **% w/w** |
|---|---|---|
| Guar Gum | 21 CFR 184.1339 / organic | 30 |
| Gum Arabic | FCC / organic | 30 |
| Agave Powder | USDA/NOP / organic | 30 |
| Rice Concentrate | FCC / organic | 10 |

### Example 13

| **Excipient:** | **Grades:** | **% w/w** |
|---|---|---|
| Guar Gum | 21 CFR 184.1339 / organic | 30 |
| Gum Arabic | FCC / organic | 30 |
| Honey Powder | organic | 30 |
| Rice Concentrate | FCC / organic | 10 |

### Example 14

| **Excipient:** | **Grades:** | **% w/w** |
|---|---|---|
| Guar Gum | 21 CFR 184.1339 / organic | 30 |
| Gum Arabic | FCC / organic | 30 |
| Isolated pea protein | organic | 30 |
| Rice Concentrate | FCC / organic | 10 |

### Example 15:

The above binder formulations were used to prepare tablets as described under Binder Formulation 1-3. The tablets were investigated with respect to tablet hardness. The different investigated formulations were compressed on a FlexiTab^{®} S, single station tablet press in laboratory scale. After the compaction process the tablets were tested on tablet hardness according to Ph. Eur. 2.9.8 with PTB111E (tablet hardness tester).

Used Equipment:

| **Equipment** | **Manufacturer** | **Specifcation** |
|---|---|---|
| Tablet press | Röltgen Marking Systems GmbH | -FlexiTab^{®} S |
| | | -TSM/Euro B/D compliant |
| Punch (upper- and lower punch) | Adamus SA | 08/13, Ø10, 03 |
| Punch die | Adamus SA | 08/13, Ø10, 03 |
| Tablet hardness tester | PharmaTest Gerätebau GmbH | -PTB111 E |
| | | -Ph. Eur. 2.9.8 compliant |
| Free-fall mixer | Glass GmbH & Co. KG | -LPM 20 |

Used Parameters:

| | |
|---|---|
| **Dosage form** | tablet |
| **Shape** | biconvex, round |
| **Diameter** | 10 mm |
| **Compression force** | 25 kN |
| **Filling depth** | 8 mm |

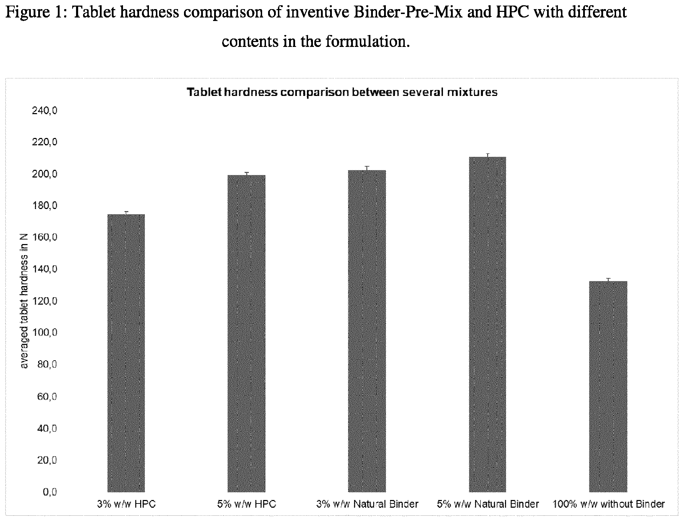

Figure 1 illustrates the results of the tablet hardness investigation. Surprisingly, it is possible to provide tablet coating which provide higher tablet hardness when compared to state of the art binding compositions using same amounts of binder.

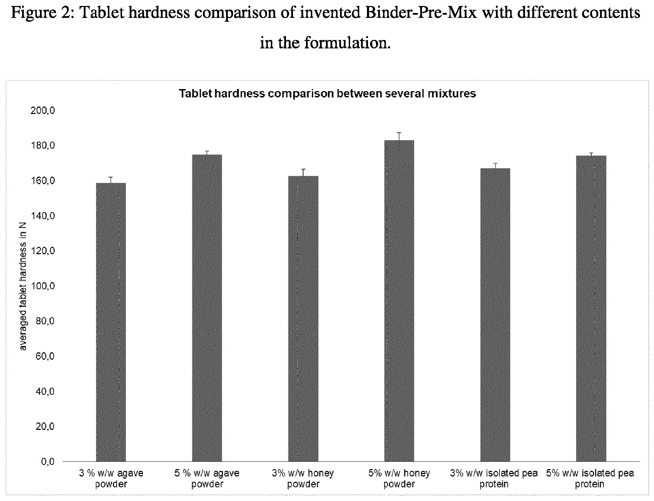

Figure 2 illustrates the Tablet hardness comparison of Binder-Pre-Mix composition according to the present invention with different contents of honey powder, agave powder, or isolated pea protein in the formulation, which were used in lieu of pullulan.

## Claims

1. A natural binder composition which comprises a blend of components a. to d. selected from: a. of 5% to 65% by weight guar gum, xanthan gum or a combination thereof; b. of 5% to 65% by weight gum arabic; of 5% to 65% by weight pullulan, honey powder, agave powder, or isolated pea protein or any combination of the foregoing; and d. 1% to 15% by weight natural plant fibers, all weight percentages being based on the dry weight of the binder composition

2. A natural binder composition which consists of a blend of components a. to d. selected from: a. of 5% to 65% by weight guar gum, xanthan gum or a combination thereof; b. of 5% to 65% by weight gum arabic; of 5% to 65% by weight pullulan, honey powder, agave powder, or isolated pea protein or any combination of the foregoing; and d. 1% to 15% by weight natural plant fibers, all weight percentages being based on the dry weight of the binder composition.

3. The natural binder composition of any of claims 1 or 2, wherein the natural plant fibers are selected from apple fiber, cellulose powder, dehydrated fruit powder, rice powder or rice extract and mixtures thereof.

4. The natural binder composition of claim 3, wherein dehydrated fruit powders are selected from banana powder, apple powder, pear powder, blueberry powder, cherry powder, cranberry powder, grape powder, peach powder, strawberry powder, raspberry powder and mixtures of the foregoing.

5. The natural binder composition of any of claims 1 to 4, wherein the component (c.) selected from the group of pullulan, honey powder, agave powder, or isolated pea protein is present in an amount ranging from 20% to 40% by dry weight of the composition.

6. The natural binder composition of any of claims 1 to 5, wherein the component (a.) selected from guar gum or xanthan gum is present in an amount ranging from 20% to 40% by dry weight of the composition.

7. The natural binder composition of any of claims 1 to 6, wherein (b.) gum arabic is present in an amount ranging from 20% to 40% by dry weight of the composition.

8. The natural binder composition of any of claims 1 to 7, wherein (d.) natural plant fiber is present in an amount ranging from 2% to 8% by dry weight of the composition.

9. A method for producing a natural binder composition wherein a blend of components a. to d. selected from: a. of 5% to 65% by weight guar gum, xanthan gum or a combination thereof; b. of 5% to 65% by weight gum arabic; of 5% to 65% by weight pullulan, honey powder, agave powder, or isolated pea protein or any combination of the foregoing; and d. 1% to 15% by weight natural plant fibers, all weight percentages being based on the dry weight of the binder composition, is provided and physically blended until a homogenous mixture is attained.

10. The method according to claim 9, wherein the homogenous mixture is further granulated.

11. A use of the natural binder composition according to any of claims 1 to 8 for producing a solid dosage form formulation.

12. The use of claim 11, wherein the solid dosage form formulation is a tablet, granules or pellets.

13. A solid dosage form formulation comprising the binder composition of any of claims 1 to 8 in an amount ranging from 1% to 60%, based on the total weight of the tablet in the tablet formulation (on a dry weight basis).

14. A solid dosage form formulation comprising the binder composition of any of claims 1 to 8 in an amount ranging from 1% to 40% w/w, based on the total weight of the tablet in the tablet formulation (on a dry weight basis).

15. A solid dosage form formulation comprising the binder composition of any of claims 1 to 8 in an amount ranging from 2% to 20% w/w, based on the total weight of the tablet in the tablet formulation (on a dry weight basis).

## Patentansprüche

1. Natürliche Bindemittelzusammensetzung, die eine Mischung der Komponenten a. bis d. umfasst, die ausgewählt sind aus: a. 5 bis 65 Gew.-% Guargummi, Xanthangummi oder einer Kombination davon; b. 5 bis 65 Gew.-% Gummi arabicum; 5 bis 65 Gew.-% Pullulan, Honigpulver, Agavenpulver oder isoliertes Erbsenprotein oder eine beliebige Kombination der vorgenannten; und d. 1 bis 15 Gew.-% natürliche Pflanzenfasern, wobei alle Gewichtsprozente auf das Trockengewicht der Bindemittelzusammensetzung bezogen sind.

2. Natürliche Bindemittelzusammensetzung, die aus einer Mischung der Komponenten a. bis d. besteht, die ausgewählt sind aus: a. 5 bis 65 Gew.-% Guargummi, Xanthangummi oder einer Kombination davon; b. 5 bis 65 Gew.-% Gummi arabicum; 5 bis 65 Gew.-% Pullulan, Honigpulver, Agavenpulver oder isoliertes Erbsenprotein oder eine beliebige Kombination der vorgenannten; und d. 1 bis 15 Gew.-% natürliche Pflanzenfasern, wobei alle Gewichtsprozente auf das Trockengewicht der Bindemittelzusammensetzung bezogen sind.

3. Natürliche Bindemittelzusammensetzung nach einem der Ansprüche 1 oder 2, wobei die natürlichen Pflanzenfasern aus Apfelfasern, Zellulosepulver, Trocken-Fruchtpulver, Reispulver oder Reisextrakt und Mischungen davon ausgewählt sind.

4. Natürliche Bindemittelzusammensetzung nach Anspruch 3, wobei die Trocken-Fruchtpulver ausgewählt sind aus Bananenpulver, Apfelpulver, Birnenpulver, Heidelbeerpulver, Kirschpulver, Preiselbeerpulver, Traubenpulver, Pfirsichpulver, Erdbeerpulver, Himbeerpulver und Mischungen der vorgenannten.

5. Natürliche Bindemittelzusammensetzung nach einem der Ansprüche 1 bis 4, wobei die Komponente (c), die aus der Gruppe Pullulan, Honigpulver, Agavenpulver oder isoliertes Erbsenprotein ausgewählt ist, in einer Menge von 20 bis 40 % des Trockengewichts der Zusammensetzung vorhanden ist.

6. Natürliche Bindemittelzusammensetzung nach einem der Ansprüche 1 bis 5, wobei die Komponente (a.), ausgewählt aus Guarkernmehl oder Xanthangummi, in einer Menge von 20 bis 40 % des Trockengewichts der Zusammensetzung vorhanden ist.

7. Natürliche Bindemittelzusammensetzung nach einem der Ansprüche 1 bis 6, wobei (b.) Gummi arabicum in einer Menge von 20 % bis 40 % des Trockengewichts der Zusammensetzung vorhanden ist.

8. Natürliche Bindemittelzusammensetzung nach einem der Ansprüche 1 bis 7, wobei (d.) natürliche Pflanzenfasern in einer Menge von 2 % bis 8 % des Trockengewichts der Zusammensetzung vorhanden sind.

9. Verfahren zur Herstellung einer natürlichen Bindemittelzusammensetzung, bei dem eine Mischung der Komponenten a. bis d., ausgewählt aus: a. 5 bis 65 Gew.-% Guargummi, Xanthangummi oder einer Kombination davon; b. 5 bis 65 Gew.-% Gummi arabicum; 5 bis 65 Gew.-% Pullulan, Honigpulver, Agavenpulver oder isoliertes Erbsenprotein oder eine beliebige Kombination der vorgenannten; und d. 1 bis 15 Gew.-% natürliche Pflanzenfasern, wobei alle Gewichtsprozente auf das Trockengewicht der Bindemittelzusammensetzung bezogen sind, bereitgestellt und physikalisch vermischt wird, bis eine homogene Mischung erreicht ist.

10. Verfahren nach Anspruch 9, wobei das homogene Gemisch weiter granuliert wird.

11. Verwendung der natürlichen Bindemittelzusammensetzung nach einem der Ansprüche 1 bis 8 zur Herstellung einer festen Dosierungsformulierung.

12. Verwendung nach Anspruch 11, wobei die feste Dosierungsformulierung eine Tablette, ein Granulat oder Pellets ist.

13. Feste Dosierungsformulierung, umfassend die Bindemittelzusammensetzung nach einem der Ansprüche 1 bis 8 in einer Menge von 1% bis 60%, bezogen auf das Gesamtgewicht der Tablette in der Tablettenformulierung (auf Trockengewichtsbasis) .

14. Feste Dosierungsformulierung, umfassend die Bindemittelzusammensetzung nach einem der Ansprüche 1 bis 8 in einer Menge im Bereich von 1% bis 40% w/w, bezogen auf das Gesamtgewicht der Tablette in der Tablettenformulierung (auf Trockengewichtsbasis) .

15. Feste Dosierungsformulierung, umfassend die Bindemittelzusammensetzung nach einem der Ansprüche 1 bis 8 in einer Menge im Bereich von 2 % bis 20 % w/w, bezogen auf das Gesamtgewicht der Tablette in der Tablettenformulierung (auf Trockengewichtsbasis) .

## Revendications

1. Composition naturelle de liant, comprenant un mélange de composants (a) à (d) choisis parmi :
a) de 5 à 65 % en poids de gomme de guar, de gomme xanthane ou d'une combinaison de celles-ci,
b) de 5 à 65 % en poids de gomme arabique,
c) de 5 à 65 % en poids de pullulane, de miel en poudre, de poudre d'agave ou de protéine de pois isolée, ou de n'importe quelle combinaison de ces composants,
d) et de 1 à 15 % en poids de fibres végétales naturelles,
ces pourcentages pondéraux étant tous rapportés au poids à sec de la composition de liant.

2. Composition naturelle de liant, constituée d'un mélange de composants (a) à (d) choisis parmi :
a) de 5 à 65 % en poids de gomme de guar, de gomme xanthane ou d'une combinaison de celles-ci,
b) de 5 à 65 % en poids de gomme arabique,
c) de 5 à 65 % en poids de pullulane, de miel en poudre, de poudre d'agave ou de protéine de pois isolée, ou de n'importe quelle combinaison de ces composants,
d) et de 1 à 15 % en poids de fibres végétales naturelles,
ces pourcentages pondéraux étant tous rapportés au poids à sec de la composition de liant.

3. Composition naturelle de liant, conforme à la revendication 1 ou 2, dans laquelle les fibres végétales naturelles sont choisies parmi des fibres de pomme, de la cellulose en poudre, des fruits déshydratés en poudre, de la poudre de riz ou de l'extrait de riz, et leurs mélanges.

4. Composition naturelle de liant, conforme à la revendication 3, dans laquelle les fruits déshydratés en poudre sont choisis parmi de la poudre de banane, de la poudre de pomme, de la poudre de poire, de la poudre de myrtille, de la poudre de cerise, de la poudre de canneberge, de la poudre de raisin, de la poudre de pêche, de la poudre de fraise, de la poudre de framboise, et leurs mélanges.

5. Composition naturelle de liant, conforme à l'une des revendications 1 à 4, dans laquelle le composant (c), choisi dans l'ensemble formé par du pullulane, du miel en poudre, de la poudre d'agave ou de la protéine de pois isolée, se trouve en une quantité représentant de 20 à 40 % du poids à sec de la composition.

6. Composition naturelle de liant, conforme à l'une des revendications 1 à 5, dans laquelle le composant (a), choisi parmi de la gomme de guar et de la gomme xanthane, se trouve en une quantité représentant de 20 à 40 % du poids à sec de la composition.

7. Composition naturelle de liant, conforme à l'une des revendications 1 à 6, dans laquelle le composant (b), de la gomme arabique, se trouve en une quantité représentant de 20 à 40 % du poids à sec de la composition.

8. Composition naturelle de liant, conforme à l'une des revendications 1 à 7, dans laquelle le composant (d), des fibres végétales naturelles, se trouve en une quantité représentant de 2 à 8 % du poids à sec de la composition.

9. Procédé de production d'une composition naturelle de liant, dans lequel un mélange de composants (a) à (d) choisis parmi :
a) de 5 à 65 % en poids de gomme de guar, de gomme xanthane ou d'une combinaison de celles-ci,
b) de 5 à 65 % en poids de gomme arabique,
c) de 5 à 65 % en poids de pullulane, de miel en poudre, de poudre d'agave, ou de protéine de pois isolée, ou de n'importe quelle combinaison de ces composants,
d) et de 1 à 15 % en poids de fibres végétales naturelles,
ces pourcentages pondéraux étant tous rapportés au poids à sec de la composition de liant, est préparé et physiquement mélangé jusqu'à l'obtention d'un mélange homogène.

10. Procédé conforme à la revendication 9, dans lequel le mélange homogène est ensuite granulé.

11. Utilisation d'une composition naturelle de liant, conforme à l'une des revendications 1 à 8, pour produire une formulation de forme posologique solide.

12. Utilisation conforme à la revendication 11, dans laquelle la formulation de forme posologique solide est un comprimé, des granules ou des pilules.

13. Formulation de forme posologique solide, comprenant une composition de liant conforme à l'une des revendications 1 à 8 en une proportion valant de 1 à 60 %, rapportée au poids total du comprimé dans la formulation de comprimé (en poids à sec).

14. Formulation de forme posologique solide, comprenant une composition de liant conforme à l'une des revendications 1 à 8 en une proportion valant de 1 à 40 % pds/pds, rapportée au poids total du comprimé dans la formulation de comprimé (en poids à sec).

15. Formulation de forme posologique solide, comprenant une composition de liant conforme à l'une des revendications 1 à 8 en une proportion valant de 2 à 20 % pds/pds, rapportée au poids total du comprimé dans la formulation de comprimé (en poids à sec).
